Europäisches Patentamt

European Patent Office

Office européen des brevets

(11) Veröffentlichungsnummer: **0 080 098**
**B1**

(12) **EUROPÄISCHE PATENTSCHRIFT**

(45) Veröffentlichungstag der Patentschrift:
**12.09.84**

(51) Int. Cl.³: **C 07 C 19/045,** C 07 C 17/02

(21) Anmeldenummer: **82110276.1**

(22) Anmeldetag: **08.11.82**

(54) **Verfahren zur Herstellung von 1,2-Dichlorethan.**

(30) Priorität: **21.11.81 DE 3146246**

(43) Veröffentlichungstag der Anmeldung:
**01.06.83 Patentblatt 83/22**

(45) Bekanntmachung des Hinweises auf die Patenterteilung:
**12.09.84 Patentblatt 84/37**

(84) Benannte Vertragsstaaten:
**BE CH DE FR GB IT LI NL SE**

(56) Entgegenhaltungen:
**EP - A - 0 001 417**
**EP - A - 0 026 349**
**DE - A - 2 427 045**
**GB - A - 1 231 127**

(73) Patentinhaber: **HOECHST AKTIENGESELLSCHAFT,**
**Postfach 80 03 20, D-6230 Frankfurt am Main 80 (DE)**

(72) Erfinder: **Hundeck, Joachim, Dr., Argelanderstrasse 135,**
**D-5300 Bonn 1 (DE)**
Erfinder: **Hennen, Hans, Kendenicherstrasse 86,**
**D-5030 Hürth (DE)**

## Beschreibung

Die Erfindung betrifft ein Verfahren zur Herstellung von 1,2-Dichlorethan durch Umsetzung von Ethylen und Chlor in flüssigem 1,2-Dichlorethan in Gegenwart eines hierfür üblichen Katalysators, wobei die bei der Reaktion entstehende Reaktionswärme nutzbringend verwendet und die unerwünschte Bildung höher chlorierter Produkte, wie Tri-, Tetra- und Pentachlorethan, im Reaktor weitgehend vermieden sowie die Kumulierung derartiger Produkte in der Reaktionszone verhindert wird.

Die Chlorierung von Olefinen mittels Chlor ist bekanntlich eine exotherme Reaktion. Im Falle der Chlorierung von Ethylen mit Chlor beträgt die Wärmetönung 2200 kJ pro Kilogramm 1,2-Dichlorethan. Bei der Produktion einer Tonne 1,2-Dichlorethan fällt somit eine Wärmemenge an, die ausreicht, um etwa 1 Tonne Dampf zu erzeugen. Bei den bekannten Verfahren zur Herstellung von Dichlorethan wurde die Reaktionswärme entweder durch Kühlung des Reaktors abgeführt oder sie wurde teilweise zur unmittelbaren Verdampfung und Abtreibung des bei der Reaktion entstandenen Dichlorethans aus dem Reaktionsgemisch bzw. dem Reaktor und in einzelnen Fällen auch bereits zur auschliesslichen Rektifizierung von nach anderer Verfahrensweise hergestelltem 1,2-Dichlorethan mehr oder weniger vollständig genutzt.

Eine kombinierte Abführung der Reaktionswärme bei der Chlorierung von Ethylen mit Chlor wird ferner beispielsweise bei dem in der DE-PS 1543108 beschriebenen Verfahren praktiziert. Hierbei ist der zur Durchführung der Reaktion vorgesehene Eisenreaktor mit einer Kühlvorrichtung versehen, welche mit Kühlwasser beaufschlagt ist, um bei der Reaktion entstehende Wärme abzuführen und die für die Umsetzung vorbestimmte Reaktionstemperatur von 50-70°C einhalten zu können. Die Einhaltung der unterhalb des Siedepunktes von 1,2-Dichlorethan liegenden Reaktionstemperatur wird während der Reaktion so geregelt, dass das gebildete 1,2-Dichlorethan in dampfförmigen Zustand kontinuierlich aus dem Reaktionsraum entfernt wird. Bei dieser Verfahrensweise findet zwar keine Kumulierung höher chlorierter Produkte im Reaktor statt, doch werden dabei 3,3% Trichlorethan gebildet und die Reaktionswärme kann nicht genutzt werden, weil das kondensierte 1,2-Dichlorethan noch von dem erwähnten Nebenprodukt befreit werden muss.

Ein im Prinzip mit dem Verfahren der DE-PS 1543108 übereinstimmendes Verfahren wird in der DE-OS 2935884 offenbart, wobei die charakteristischen Merkmale letztgenannten Verfahrens darin bestehen, dass die Reaktorfüllung während der Reaktion über eine mit dem Reaktor in Verbindung stehende Ringleitung umgewälzt wird und die am Kopf des Reaktors abziehenden dampfförmigen Produkte in einer Rektifizierkolonne unter Gewinnung von 1,2-Dichlorethan getrennt werden. Höher siedende Nebenprodukte werden dabei nachteiligerweise vom Sumpf der Rektifizierkolonne in den Reaktor zurückgeleitet, wodurch in diesem Falle eine separate Aufarbeitung diskontinuierlich entnommener Mengen Reaktionssumpfprodukt erforderlich ist und die Reaktionswärme nur teilweise genutzt werden kann.

Schliesslich besteht eine weitere Verfahrensweise zur Herstellung von Ethylenchlorid gemäss DE-OS 2427045 darin, dass man

a) Ethylen und Chlor in eine unter erhöhtem Druck stehende Reaktionszone, welche ein umlaufendes flüssiges Medium mit einem Gehalt an chlorierten Kohlenwasserstoffen mit zwei Kohlenstoffatomen oder Gemischen dieser chlorierten Kohlenwasserstoffe enthält, das bei einer Temperatur unterhalb der Verdampfungstemperatur des Mediums bei dem in der Reaktionszone herrschenden Druck gehalten wird, einleitet, wobei rohes flüssiges Ethylendichlorid gebildet wird;

b) das rohe flüssige Ethylendichlorid mit dem umlaufenden Medium zu einer unter niedrigerem Druck stehenden Zone führt, wobei man Druck und Temperatur dieser Zone auf Werten hält, bei denen das unreine Ethylendichlorid durch die bei der Umsetzung von Chlor und Ethylen freiwerdende Reaktionswärme verdampft, und

c) das dampfförmige unreine Ethylendichlorid in eine Rektifikationszone einleitet und mit Hilfe der bei der Reaktion von Chlor und Ethylen freiwerdenden Reaktionswärme rektifiziert, wobei das gereinigte Ethylendichlorid aus der Rektifikationszone abgezogen wird, während das Sumpfprodukt der Rektifizierkolonne in die Zone niedrigeren Drucks zurückgeführt und mit dem umlaufenden Medium vereinigt wird.

Die Rückführung des Sumpfproduktes in den Reaktor ist insofern nachteilig, als das umlaufende flüssige Medium mit höher siedenden Chlorierungsprodukten angereichert wird, welche aus letzterem entfernt werden müssen. Gemäss Beispiel 4 der DE-OS 2427045 beträgt der Anteil an 1,1,2-Trichlorethan im umlaufenden Medium etwa 60%. Dies bedeutet, dass die bekannte Chlorierungsreaktion unter Bildung erheblicher Anteile an unerwünschten Nebenprodukten verläuft.

Es bestand somit die Aufgabe, die bekannten Verfahrensweisen in dem Masse zu verbessern, dass die Bildung von Nebenprodukten weitgehend vermieden und die bei der Reaktion anfallende Reaktionswärme optimal genutzt wird. Diese Aufgabe wird bereits in hohem Masse durch das Verfahren gemäss EP-A1-75.742 (Priorität 21.09.81; Veröffentlichungstag 06.04.83) gelöst. Gegenstand dieser Patentschrift ist ein Verfahren zur Herstellung von 1,2-Dichlorethan durch Umsetzung von Ethylen und Chlor in einer Reaktionszone, welche ein umlaufendes flüssiges Medium mit einem Gehalt an chlorierten Kohlenwasserstoffen mit zwei Kohlenstoffatomen enthält, bei einer Temperatur unterhalb der Verdampfungstemperatur des Mediums bei dem in der Reaktionszone herrschenden Druck und in Gegenwart eines üblichen Katalysators zur Chlorübertragung und gegebenenfalls eines Inhibitors zur Verringerung der Nebenproduktbildung, unter Bildung von rohem 1,2-Dichlorethan, welches aus der Reaktionszone abgezogen und in einer anschliessenden separaten Fraktionierkolonne gereinigt wird, welches dadurch gekennzeichnet ist, dass man

a) etwa äquimolare Mengen von Ethylen und Chlor in das umlaufende flüssige Medium einleitet und nach intensiver Durchmischung in einer Mischzone das Gemisch in einer Reaktionszone bei einer Temperatur von etwa 75-200°C und einem Druck von etwa 1-15 bar zur Reaktion bringt, wobei die mittlere Verweilzeit des Reaktionsgemisches in der Misch- und Reaktionszone etwa 1-15 Stunden beträgt;

b) aus der Reaktionszone einen Teil des flüssigen Reaktionsgemisches abzieht und letzteren in zwei Teilströme aufteilt, wobei ein Teilstrom zur Abgabe von Wärmeenergie einen Wärmeaustauscher passiert und danach mit verminderter Temperatur in die Misch- und Reaktionszone zurückfliesst, während der zweite Teilstrom einem Entspannungsgefäss zugeführt wird, in welchem eine adäquate Menge des in der Reaktionszone gebildeten Reaktionsproduktes sowie gegebenenfalls ein Anteil nach anderer Verfahrensweise hergestelltes und der Reaktionszone zugeführtes 1,2-Dichlorethan aus dem zweiten Teilstrom verdampft, wobei die Dämpfe in eine Fraktionierkolonne eingeleitet werden, während der nicht verdampfte, flüssige Anteil des zweiten Teilstromes in die Misch- und Reaktionszone des umlaufenden flüssigen Mediums zurückkehrt, und

c) aus den in die Fraktionierkolonne eingeleiteten Dämpfen das 1,2-Dichlorethan destillativ unter Verwendung eines Teiles der im Wärmeaustauscher übertragenen Wärmeenergie abtrennt und letzteres über den Kopf der Kolonne abzieht, wobei im Sumpf der Kolonne höher chlorierte Produkte anfallen, die abgezogen und separat aufgearbeitet werden.

Gegenstand der Erfindung ist nunmehr eine bevorzugte Ausführungsform des Verfahrens gemäss EP-AI-75.742, indem die Durchführung des Verfahrens in einem Doppelschlaufenreaktor praktiziert wird.

Das erfindungsgemäss verbesserte Verfahren zur Herstellung von 1,2-Dichlorethan durch Umsetzung von Ethylen und Chlor in einer Reaktionszone, welche ein umlaufendes flüssiges Medium mit einem Gehalt an chlorierten Kohlenwasserstoffen mit zwei Kohlenstoffatomen sowie einen zur Chlorübertragung üblichen Katalysator und gegebenenfalls einen Inhibitor zur Verringerung der Nebenproduktbildung enthält, wobei man

a) etwa äquimolare Mengen von Ethylen und Chlor in das umlaufende flüssige Medium einleitet, mischt und das Gemisch in einer Reaktionszone bei einer Temperatur von etwa 75-200°C , einem Druck von etwa 1-15 bar und einer mittleren Verweilzeit des Gemisches in der Misch- und Reaktionszone von etwa 1-15 Stunden zur Reaktion bringt, wobei man

b) aus der Reaktionszone einen Teil des flüssigen Reaktionsgemisches abzieht und letzteren in zwei Teilströme aufteilt, wobei ein Teilstrom zur Abgabe von Wärmeenergie einen Wärmeaustauscher passiert und danach mit verminderter Temperatur in die Misch- und Reaktionszone zurückfliesst, während der zweite Teilstrom einem Entspannungsgefäss zugeführt wird, in welchem eine adäquate Menge des in der Reaktionszone gebildeten Reaktionsproduktes aus dem zweiten Teilstrom verdampft, wobei die Dämpfe in eine Fraktionierkolonne eingeleitet werden und das 1,2-Dichlorethan destillativ abgetrennt wird, während der nicht verdampfte, flüssige Anteil des zweiten Teilstromes in die Misch- und Reaktionszone des umlaufenden flüssigen Mediums zurückkehrt, ist dadurch gekennzeichnet, dass man die Herstellung des 1,2-Dichlorethans in einem Doppelschlaufenreaktor durchführt, derart, dass man

A) über die Leitung (1) Ethylen und über die Leitung (2) Chlorgas in den Aufstiegsteil der Schlaufe (I) unterhalb der im Aufstiegsteil befindlichen Mischzone (3) einleitet und in dem in der Schlaufe (I) umlaufenden flüssigen Medium fein verteilt, wonach man die Reaktionskomponenten in der an die Mischzone (3) sich anschliessenden Reaktionszone (4) bzw. der Verweilzone (5) umsetzt; dass man

B) aus der Schlaufe (I) zwei Teilströme des Reaktionsgemisches abzieht, wovon ein Teilstrom zur Abgabe von Wärmeenergie über die Zuführungsleitung (8) den Wärmeaustauscher (10) passiert und anschliessend über die Leitung (9) in den Abstiegsteil der Schlaufe (I) zurückkehrt, während der zweite Teilstrom der mit der Schlaufe (I) integrierend verbundenen Schlaufe (II) sowie einer im Schlaufenkreislauf (II) befindlichen Entspannungszone (6) zuströmt, in welcher eine adäquate Menge des in der Reaktionszone (4) gebildeten Reaktionsproduktes aus dem zweiten Teilstrom verdampft, wobei die Dämpfe über die Abzugsleitung (7) der Fraktionierkolonne zugeführt werden, während der nicht verdampfte, flüssige Anteil des zweiten Teilstromes über den Abstiegsteil der Schlaufe (II) in die Mischzone (3) bzw. Reaktionszone (4) der Schlaufe (I) zurückkehrt.

Ein weiteres Merkmal des Verfahrens der Erfindung besteht darin, dass man im Reaktionsgemisch enthaltene inerte Gase oder leichtsiedende Kohlenwasserstoffe, wie Ethylchlorid, aus der Verweilzone (5) über die Leitung (11) oder aus dem Abstiegsteil der Schlaufe (I) über die Leitung (12) abzieht.

Im einzelnen ist zu dem Verfahren der Erfindung noch folgendes zu bemerken:

Die Reaktionspartner Ethylen und Chlor können durch inerte Gase verdünnt sein. Chlor kann in flüssiger Form oder als Gas in die Mischzone eingebracht werden, jedoch ist es zweckmässig, flüssiges Chlor vor Eintritt in den Reaktor mit Hilfe eines Teiles der Reaktionsenthalpie in einem Wärmeaustauscher zu verdampfen. Als Katalysator empfiehlt sich die Verwendung von Eisen-III-chlorid und als Inhibitor zur Vermeidung der Nebenproduktbildung vorzugsweise Sauerstoff.

Der Umlauf des flüssigen Mediums im Reaktor wird nach dem Thermosiphon- bzw. Mammutpumpenprinzip bewirkt. Die Umlaufgeschwindigkeit des flüssigen Mediums soll in der Mischzone nicht kleiner als 0,1 m/sec sein. Der Umlauf zur Produktverdampfung in der Entspannungszone erfolgt ebenfalls nach dem Thermosiphonprinzip. Der Wärmetauscher kann in die Produktkreisläufe integriert oder mittels Pumpe separat beschickt werden.

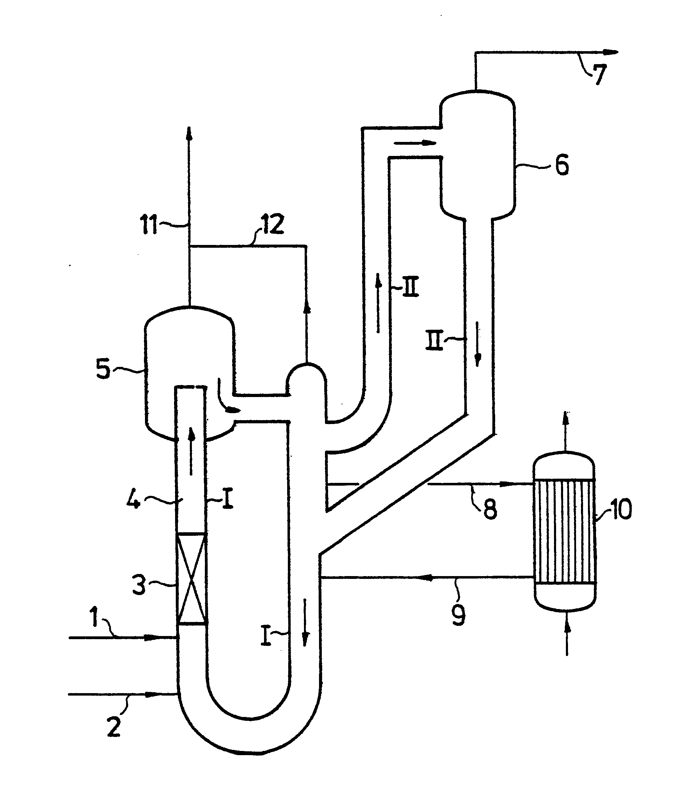

Das Verfahren der Erfindung sei im folgenden in Verbindung mit der Zeichnung näher erläutert.

Ein Doppelschlaufenreaktor mit den Schlaufenkreisläufen I und II wird zunächst mit flüssigem 1,2-Dichlorethan beschickt und letzteres durch Einleiten von Ethylen über die Leitung 1 sowie Chlorgas über die Leitung 2 nach dem Mammutpumpenprinzip in Zirkulation versetzt. Nach Eintritt der Reaktion des Ethylens mit dem Chlorgas, die in der mit Füllkörpern beschickten Mischzone 3 beginnt und in der Reaktionszone 4 sowie in der Verweilzone 5 vervollständigt wird, entsteht ein zusätzlicher Auftrieb im Aufstiegsteil der Schlaufe I, bedingt durch die freiwerdende Reaktionswärme. Die Temperatur im Schlaufenkreislauf I liegt etwas niedriger als die Siedetemperatur des 1,2-Dichlorethans bei dem im Reaktor herrschenden Druck. Eventuell in der Schlaufe I vorhandene inerte Gase werden über die Leitungen 11 und 12 abgezogen und in einem, in der Zeichnung nicht dargestellten Kühler zur Kondensation von mitgeführten 1,2-Dichlorethandämpfen gekühlt. Die nicht kondensierten Gase werden abgeblasen und in bekannter Weise aufgearbeitet. Durch Regulierung des Inertgasstromes hinsichtlich der abgeblasenen Menge kann in der Schlaufe I der gewünschte Druck eingestellt werden.

Zur Gewinnung des im Schlaufenkreislauf I produzierten 1,2-Dichlorethans werden aus dem Flüssigkeitsstrom zwei Teilströme abgezweigt, wobei ein Teilstrom zur Abgabe von Wärmeenergie über die Zuführungsleitung 8 den Wärmeaustauscher 10 passiert und anschliessend über die Leitung 9 in den Abstiegteil der Schlaufe I zurückkehrt, während der zweite Teilstrom der mit der Schlaufe I integrierend verbundenen Schlaufe II sowie einer im Schlaufenkreislauf II befindlichen Entspannungszone 6 zuströmt, in welcher eine adäquate Menge des in der Reaktionszone 4 gebildeten Reaktionsproduktes aus dem zweiten Teilstrom verdampft, wobei die Dämpfe über die Abzugsleitung 7 der in der Zeichnung nicht dargestellten Fraktionierkolonne zugeführt werden, während der nicht verdampfte, flüssige Anteil des zweiten Teilstromes über den Abstiegteil der Schlaufe II in die Mischzone 3 bzw. Reaktionszone 4 der Schlaufe I zurückkehrt. Im Reaktionsgemisch enthaltene inerte Gase oder leichtsiedende Kohlenwasserstoffe, wie Ethylchlorid, werden aus der Verweilzone 5 über die Leitung 11 oder aus dem Abstiegteil der Schlaufe I über die Leitung 12 abgezogen.

Das Verfahren der Erfindung zeichnet sich durch die gleichen Vorteile aus, wie sie dem Verfahren gemäss EP-A1-75.742 eigen sind. Sie resultieren aus der kontinuierlichen Entnahme eines Teiles des flüssigen Reaktorinhaltes und dessen Aufteilung in zwei Teilströme, wovon ein Teilstrom die laufende Rückgewinnung der Reaktionswärme ermöglicht, während die partielle Verdampfung des zweiten Teilstromes und die ausschliessliche Aufarbeitung des Dampfanteiles in der Fraktionierkolonne dazu führt, dass nur katalysatorfreies Rohdichlorethan in die Fraktionierkolonne gelangt. Mit vorgenannten Dämpfen werden aber auch die höherchlorierten Nebenprodukte der Fraktionierkolonne zugeführt, wodurch eine Kumulierung von Nebenprodukten im Schlaufenkreislauf I vermieden wird. Letztere bewirkt nämlich bei den bekannten Verfahren eine Anreicherung höherchlorierter Nebenprodukte und zwingt zur Abtrennung der Nebenprodukte aus dem Reaktor. Hierbei eintretende Katalysatorverluste müssen ergänzt werden, wodurch die Wirtschaftlichkeit dieser Verfahren beeinträchtigt wird. Durch den erfindungsgemässen Verfahrensablauf wird auch die mittlere Verweilzeit aller an der Reaktion beteiligten Stoffe unter den gegebenen Reaktionsbedingungen im Reaktor so weit vermindert, dass unerwünschte, zur Nebenproduktbildung führende Nebenreaktionen weitgehend vermieden werden.

Weiterhin ermöglicht das Verfahren der Erfindung auch den Einsatz von mit Inertgasen verunreinigten Ausgangsprodukten, die bekannterweise bei der Reinigung des Dichlorethans in der Fraktionierkolonne Schwierigkeiten bereiten. Erfindungsgemäss werden diese Inertgase bereits aus der Schlaufe I über die Inertgasleitungen 11 und 12 abgezogen, so dass sie den weiteren Aufarbeitungsprozess des Reaktionsgemisches nicht stören können.

Schliesslich erweist sich das Verfahren der Erfindung auch gegenüber dem Verfahren gemäss EP-A1-75.742 als vorteilhaft, indem die Verwendung eines Doppelschlaufenreaktors, in welchem der kontinuierliche Umlauf des Reaktionsgemisches in den Schlaufen I und II nach dem Thermosiphon- oder Mammutpumpenprinzip erfolgt, den Einsatz besonderer Pumpen zur Förderung des Reaktionsgemisches aus dem bzw. in den Reaktor erübrigt, womit auch eine Energieersparnis verbunden ist.

*Beispiel*

In einem Reaktor gemäss Zeichnung, der ca. 15 l fasst, befanden sich etwa 15 kg 1,2-Dichlorethan mit 0,1% Eisen III-chlorid als Katalysator. Über Leitung 1 wurden ca. 110 l/h Ethylen und über Leitung 2 ca. 110 l/h Chlor sowie 10 l/h Luft eingeleitet. Die Temperatur im Reaktor betrug ca. 115°C, der Druck über der Verweilzone 5 ca. 3 bar und die Druckdifferenz zwischen Verweilzone 5 und Entspannungszone 6 ca. 0,5 bar.

Aus dem Reaktorabgas, das die Verweilzone 5 über Leitung 11 verliess, wurden in einer Kühlfalle bei −30°C kondensierbare Anteile abgeschieden. Über Leitung 7 wurden 480,1 g/h Dichlorethan dampfförmig aus dem Entspannungsgefäss 6 abgezogen und kondensiert.

Zur Abführung der Reaktionswärme wurden über die Leitungen 8 und 9 etwa 200 l/h Reaktorflüssigkeit umgepumpt. Im Wärmetauscher 10 wurde die abgegebene Wärme zur Destillation bei einer Sumpftemperatur der entsprechenden Kolonne von 80-85°C genutzt.

Nach einer Laufzeit von 48 Stunden ergab die Analyse des ausgeschleusten 1,2-Dichlorethans die folgenden Werte:

| | | |
|---|---|---|
| $C_2H_5Cl$ | 0,0024 | Gew.-% |
| 1,1-EDC | <0,002 | Gew.-% |
| 1,2-EDC | 99,86 | Gew.-% |
| 1,1,2-ETC | 0,13 | Gew.-% |

## Patentansprüche

1. Ein verbessertes Verfahren zur Herstellung von 1,2-Dichlorethan durch Umsetzung von Ethylen und Chlor in einer Reaktionszone, welche ein umlaufendes flüssiges Medium mit einem Gehalt an chlorierten Kohlenwasserstoffen mit zwei Kohlenstoffatomen sowie einen zur Chlorübertragung üblichen Katalysator und gegebenenfalls einen Inhibitor zur Verringerung der Nebenproduktbildung enthält, wobei man

a) etwa äquimolare Mengen von Ethylen und Chlor in das umlaufende flüssige Medium einleitet, mischt und das Gemisch in einer Reaktionszone bei einer Temperatur von etwa 75-200°C, einem Druck von etwa 1-15 bar und einer mittleren Verweilzeit des Gemisches in der Misch- und Reaktionszone von etwa 1-15 Stunden zur Reaktion bringt, wobei man

b) aus der Reaktionszone einen Teil des flüssigen Reaktionsgemisches abzieht und letzteren in zwei Teilströme aufteilt, wobei ein Teilstrom zur Abgabe von Wärmeenergie einen Wärmeaustauscher passiert und danach mit verminderter Temperatur in die Misch- und Reaktionszone zurückfliesst, während der zweite Teilstrom einem Entspannungsgefäss zugeführt wird, in welchem eine adäquate Menge des in der Reaktionszone gebildeten Reaktionsproduktes aus dem zweiten Teilstrom verdampft, wobei die Dämpfe in eine Fraktionierkolonne eingeleitet werden und das 1,2-Dichlorethan destillativ abgetrennt wird, während der nichtverdampfte, flüssige Anteil des zweiten Teilstromes in die Misch- und Reaktionszone des umlaufenden flüssigen Mediums zurückkehrt, dadurch gekennzeichnet, dass man die Herstellung des 1,2-Dichlorethans in einem Doppelschlaufenreaktor durchführt derart, dass man

A) über die Leitung (1) Ethylen und über die Leitung (2) Chlorgas in den Aufstiegsteil der Schlaufe (I) unterhalb der im Aufstiegsteil befindlichen Mischzone (3) einleitet und in dem in der Schlaufe (I) umlaufenden flüssigen Medium fein verteilt, wonach man die Reaktionskomponenten in der an die Mischzone (3) sich anschliessenden Reaktionszone (4) bzw. der Verweilzone (5) umsetzt; dass man

B) aus der Schlaufe (I) zwei Teilströme des Reaktionsgemisches abzieht, wovon ein Teilstrom zur Abgabe von Wärmeenergie über die Zuführungsleitung (8) den Wärmeaustauscher (10) passiert und anschliessend über die Leitung (9) in den Abstiegsteil der Schlaufe (I) zurückkehrt, während der zweite Teilstrom der mit der Schlaufe (I) integrierend verbundenen Schlaufe (II) sowie einer im Schlaufenkreislauf (II) befindlichen Entspannungszone (6) zuströmt, in welcher eine adäquate Menge des in der Reaktionszone (4) gebildeten Reaktionsproduktes aus dem zweiten Teilstrom verdampft, wobei die Dämpfe über die Abzugsleitung (7) der Fraktionierkolonne zugeführt werden, während der nicht verdampfte, flüssige Anteil des zweiten Teilstromes über den Abstiegsteil der Schlaufe (II) in die Mischzone (3) bzw. Reaktionszone (4) der Schlaufe (I) zurückkehrt.

2. Verfahren nach Anspruch 1, dadurch gekennzeichnet, dass man im Reaktionsgemisch enthaltene inerte Gase oder leichtsiedende Kohlenwasserstoffe, wie Ethylchlorid, aus der Verweilzone (5) über die Leitung (11) oder aus dem Abstiegsteil der Schlaufe (I) über die Leitung (12) abzieht.

## Claims

1. An improved process for making 1,2-dichloroethane by reacting ethylene and chlorine in a reaction zone having a liquid medium containing chlorinated $C_2$-hydrocarbons circulated therein in the presence of a customary chlorination-inducing catalyst and optionally an inhibitor reducing the formation of by-products, wherein

a) approximately equimolar proportions of ethylene and chlorine are introduced into the circulated liquid medium, the whole is mixed, and the mixture is reacted in a reaction zone at a temperature of about 75-200°C under a pressure of about 1-15 bars, and during a mean sojourn time of the mixture in the mixing and reaction zone of about 1-15 hours; wherein

b) a portion of the liquid reaction mixture is removed from the reaction zone and subdivided into two partial streams, of which one is passed through a heat exchanger for dissipation of calorific energy and recycled at reduced temperature to the mixing and reaction zone, whilst the second partial stream is introduced into an expansion vessel in which a proportion adequate to the reaction product formed in the reaction zone is evaporated from the said second partial stream, the resulting vapors are introduced into a fractionating column and 1,2-dichloroethane is distillatively separated, whereas the unevaporated liquid fraction of the second partial stream is recycled into the liquid medium circulated in the mixing and reaction zone, which comprises preparing the 1,2-dichloroethane inside a double loop reactor by

A) introducing ethylene through line (1) and chlorine gas through line (2) into the ascending portion of loop (I) below the mixing zone (3) in the asending portion and finely distributing them in the liquid medium circulated through loop (I), and then reacting the reaction components in reaction zone (4) and sojourn zone (5), respectively, following mixing zone (3), and

B) removing from loop (I) two partial streams of reaction mixture, of which one is introduced through line (8) into heat exchanger (10) for dissipation of calorific energy and recycled through line (9) into the descending portion of loop (I) whilst the second partial stream is introduced into loop (II) integrally connected to loop (I) and into expansion zone (6) provided in loop cycle (II), in which a proportion of reaction product adequate to the reaction product formed in reaction zone (4) is evaporate from said second partial stream, the resulting vapors are delivered through line (7) to the fractionating column, whilst the unevaporated liquid fraction of the second partial stream is recycled through the descending portion of loop (II) into the mixing zone (3) and reaction zone (4), respectively, of loop (I).

2. Process as claimed in claim 1, wherein inert gases or low-boiling hydrocarbons, such as ethyl chloride, contained in the reaction mixture are removed from sojourn zone (5) through line (11) or from the descending portion of loop (I) through line (12).

**Revendications**

1. Procédé perfectionné pour la préparation de 1,2-dichloroéthane par réaction d'éthylène et de chlore dans une zone de réaction renfermant un milieu liquide en circulation contenant des hydrocarbures chlorés en $C_2$ ainsi qu'un catalyseur habituel déclenchant la chloration et, éventuellement, un inhibiteur diminuant la formation de produits secondaires, dans lequel

a) on introduit des quantités à peu près équimolaires d'éthylène et de chlore dans le milieu liquide en circulation, on mélange le tout et on fait réagir le mélange dans une zone de réaction à une température d'environ 75-200°C, sous une pression d'environ 1-15 bars et pendant une durée de séjour moyenne de mélange et de réaction d'environ 1-15 h, dans lequel

b) on soutire de la zone de réaction une partie du mélange de réaction liquide et on la subdivise en deux courants partiels, dont l'un est envoyé à travers un échangeur de chaleur pour la dissipation d'énergie calorifique et recyclé ensuite à température réduite dans la zone de mélange et de réaction, tandis que l'autre courant partiel est envoyé à un récipient de détente, dans lequel on évapore de ce second courant partiel une quantité adéquate du produit de réaction formé dans la zone de réaction, les vapeurs étant introduites dans une colonne de fractionnement et le 1,2-dichloroéthane étant séparé par distillation, tandis que la fraction liquide non évaporée du second courant partiel est renvoyée dans la zone de mélange et de réaction avec le milieu liquide en circulation, caractérisé en ce que l'on prépare le 1,2-dichloroéthane dans un réacteur à deux boucles,

A) en introduisant de l'éthylène par la conduite (1) et du chlore gazeux par la conduite (2) dans la partie ascendante de la boucle (I) au-dessous de la zone de mélange (3) dans la partie ascendante et en les distribuant finement dans le milieu en circulation dans la boucle (I) et en faisant réagir ensuite les composants de réaction dans la zone de réaction (4) et la zone de séjour (5) respectivement, faisant suite à la zone de mélange (3);

B) en soutirant de la boucle (I) deux courants partiels du mélange de réaction, dont l'un est envoyé par la conduite (8) dans l'échangeur de chaleur (10) pour l'évacuation de chaleur et recyclé par la conduite (9) dans la partie descendante de la boucle (I), tandis que l'autre courant partiel est envoyé dans la boucle (II) reliée intégralement à la boucle (I) et dans une zone de détente (6) agencée dans le circuit de boucle (II), dans laquelle on évapore, du second courant partiel, une quantité adéquate du produit de réaction formé dans la zone de réaction (4), les vapeurs étant envoyées par la conduite de sortie (7) à la colonne de fractionnement, tandis que la fraction liquide non évaporée du second courant partiel retourne par la partie descendante de la boucle (II) dans la zone de mélange (3) et la zone de réaction (4), respectivement faisant partie de la boucle (I).

2. Procédé selon la revendication 1, caractérisé en ce que l'on soutire des gaz inertes ou des hydrocarbures à faible point d'ébullition, tels que le chlorure d'éthyle, contenus dans le mélange de réaction, de la zone de séjour (5) par la conduite (11) ou de la partie descendante de la boucle (I) par la conduite (12).